# EUROPEAN PATENT SPECIFICATION

(11) **EP 1 849 777 B1**
(45) Date of publication and mention of the grant of the patent: **29.10.2008**
(21) Application number: 07380165.6
(22) Date of filing: 07.06.2007
(51) Int. Cl.: C07D 257/04, A61K 31/41

(54) **Process for obtaining a valsartan salt useful for obtaining valsartan**
Verfahren zur Gewinnung eines zur Gewinnung von Valsartan nützlichen Valsartan-Salzes
Procédé pour l'obtention d'un sel de valsartan utile pour l'obtention de valsartan

(43) Date of publication of application: 31.10.2007
(73) Proprietor: INKE, S.A., 08755 Castellbisbal (ES)
(72) Inventor: Huguet Clotet, Joan, 08755 Castellbisbal (ES); Dalmases Barjoan, Pere, 08755 Castellbisbal (ES)
(74) Representative: Ponti Sales, Adelaida

(56) References cited:
- EP-A1- 0 443 983
- WO-A-02/06253
- WO-A-20/05102987

## Description

### Field of the invention

The present invention relates to a new salt of Valsartan, in particular to the high-purity dilithium salt of Valsartan, useful for obtaining Valsartan with a high yield.

Valsartan is an active compound useful for manufacturing a drug for treating hypertension and/or heart failure.

### Background of the invention

On the one hand, Spanish patent ES2084801T (equivalent to EP0443983) discloses N-acyl compounds, among which is the Valsartan of formula (I).

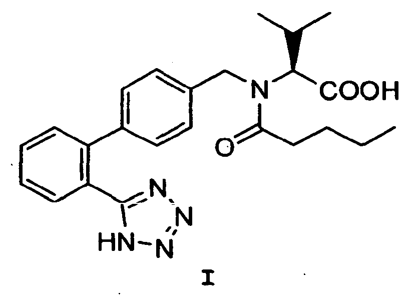

Said patent describes the preparation of Valsartan by converting a phenyl substituent (Z₁) into a tetrazole, where Z₁ is a group convertible into tetrazole and may be, among others, a halogen group. The examples of said patent describe specific cases in which Z₁ is a cyano group or a protected tetrazole ring. This is followed by deprotection of carboxyl group, where R is preferable methyl or benzyl and, if applicable, the tetrazole group, preferably protected with a trityl group, is also deprotected.

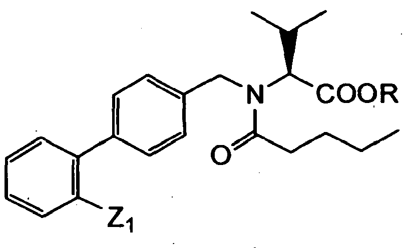

On the other hand, published Spanish patent application ES2251292 (equivalent to WO 2005/102987) discloses improvements in the process, in terms of yield, environmental protection and absence of racemization.

Specifically, said Spanish patent application ES2251292 describes introducing the tetrazole in a last synthesis step without the need to prepare it, thereby avoiding the use of sodium azide, which results in safety problems owing to its high risk of explosion, or the use of tin tributyl azide, which results in environmental problems due to the residues generated.

Said patent application discloses said introduction of the tetrazole group by means of a catalytic reaction using an organic or inorganic base capable of forming a salt with the tetrazole ring in a polar or aqueous solvent, or a mixture of water and water-miscible organic solvent. However, the examples of said application only exemplify the use of sodium hydroxide or sodium methoxide as inorganic base, and methanol or mixtures with water (ethanol/water and methanol/water) as solvent.

The process described in said Spanish patent application, in particular in Example 7 thereof, evidences the need to carry out a purification step by means of silica column chromatography following crystallisation with ethyl acetate and methylcyclohexane to obtain high-purity Valsartan, both in homogeneous catalysis and heterogeneous catalysis.

Column chromatography has problems of high consumption of solvents and generation of silica gel residues, as well as technical complications for its industrial implementation and a long consuming-time. All this involves a considerable economic cost.

### Summary description of the invention

In order to overcome the aforesaid disadvantages in the state of the art, the authors of the present invention have surprisingly found that the use of a specific salt of Valsartan is useful for obtaining Valsartan with good yields, and does not require purification by means of silica gel column chromatography to obtain said Valsartan. It thus overcomes the disadvantages relating to the use of said equipments, both from the viewpoint of the residues generated from the silica gel and the problems derived from a large consumption of solvents in a chromatography apparatus of this type, in addition to the technical complications of its industrial implementation and the long time-consuming.

Undoubtedly, there is provided a salt of Valsartan with many advantages as an intermediate product for providing Valsartan.

Thus, in a first aspect the present invention provides a dilithium salt of Valsartan, its solvates or hydrates and a polymorph thereof. In a second aspect of the invention a process for obtaining thereof is provided, and in a third aspect its use for obtaining Valsartan, an active compound useful for manufacturing a drug for treating hypertension and heart failure.

### Description of the figures

Figure 1 shows a powder X-ray diffractogram of Form I of the dilithium salt of Valsartan characterised as trihydrate.
Figure 2 shows the IR spectrum of Form I of the dilithium salt of Valsartan characterised as trihydrate.
Figure 3 shows the typical DSC of Form I of the dilithium salt of Valsartan characterised as trihydrate.

### Detailed description of the invention

In accordance with the first aspect of the present invention a dilithium salt of Valsartan of formula (I) is provided:

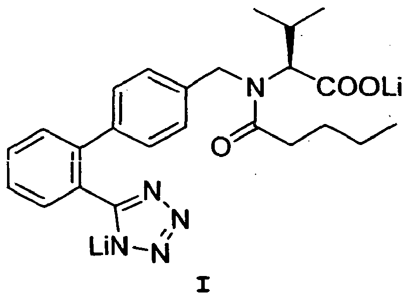

The dilithium salt of Valsartan, its polymorphs and solvates or hydrates thereof and a polymorph thereof, designated as Form I of the dilithium salt of Valsartan
characterised as trihydrate, are useful for obtaining Valsartan without the need to carry out purification by silica gel chromatography.

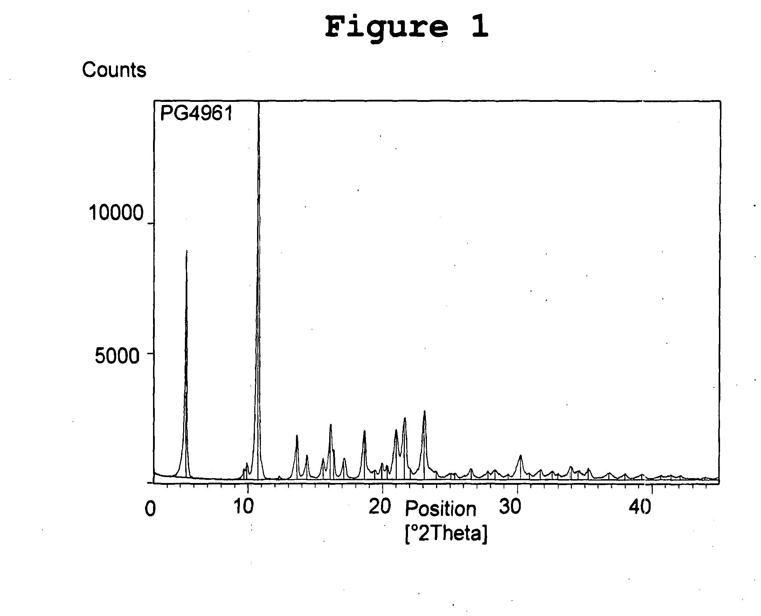

Form I of the dilithium salt of Valsartan characterised as trihydrate is characterised by a powder X-ray diffractogram like that shown in Figure 1 and Table 1 below.

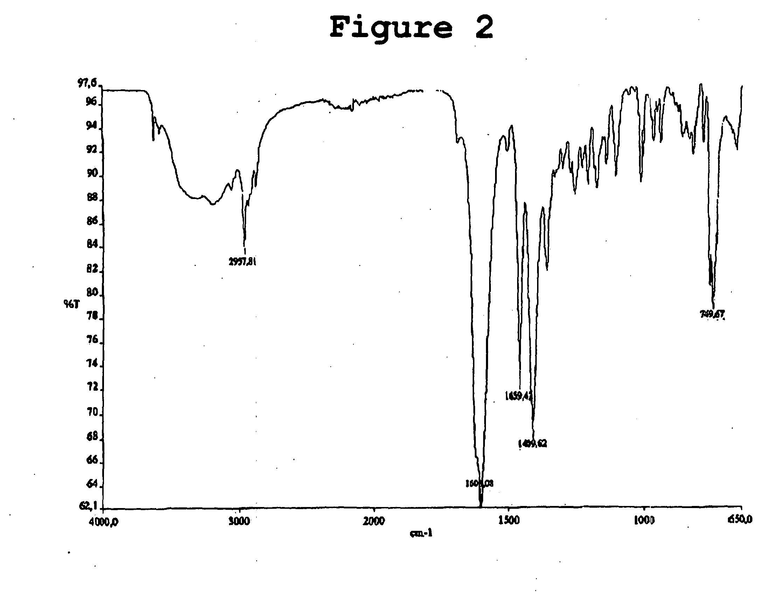

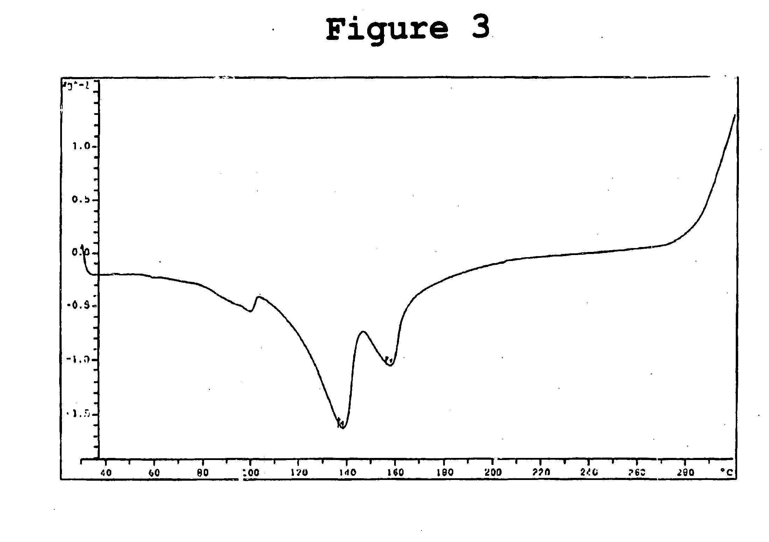

The polymorph Form I of the dilithium salt of Valsartan characterised as trihydrate is also characterised by an infrared spectrum like that shown in Figure 2 (IR (ATR): 3700-3000, 2958, 1603, 1459, 1410, 750 cm⁻¹), and a DSC (10.0°C/min) like that shown in Figure 3 with two minimums, one at 137.5°C and a second one at 157.2°C.

**Table 1**

| Peaks in ° (degrees) 2θ and d-spacing (Å) | |
|---|---|
| Pos, 2θ | d-spacing, Å |
| 5.4 | 16.4042 |
| 10.7 | 8.2357 |
| 13.6 | 6.5069 |
| 14.4 | 6.1603 |
| 15.6 | 5.6881 |
| 16.1 | 5.4943 |
| 16.4 | 5.4120 |
| 17.1 | 5.1750 |
| 18.6 | 4.7612 |
| 21.0 | 4.2288 |
| 21.6 | 4.1101 |
| 23.1 | 3.8572 |
| 30.2 | 2.9585 |

In accordance with the second aspect of the present invention, a process for obtaining said dilithium salt of Valsartan (I) with a high yield is provided. Advantageously, obtaining said dilithium salt of Valsartan or a hydrate or solvate thereof does not require purification by means of chromatography on silica gel column when preparing the Valsartan (I), which can be obtained by isolating or without isolating this intermediate.

The process for obtaining the dilithium salt of Valsartan, in accordance with the second aspect of the present invention, comprises:
i) Coupling of the intermediates **II** and **III**: 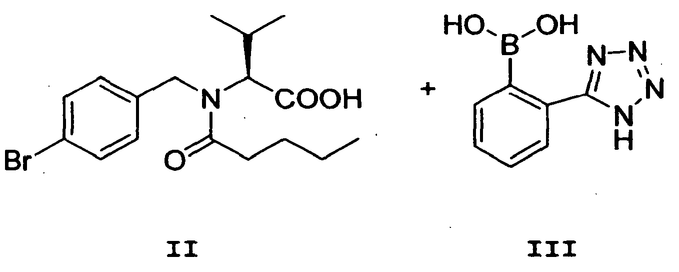
characterised in that said coupling takes place in the presence of a lithium base selected from a lithium carbonate or lithium hydroxide or a hydrate of these bases, preferably lithium hydroxide or a hydrate thereof,
in a mixture of water and water-miscible organic solvent, preferably an alcohol selected from methanol, ethanol, 1-propanol, 2-propanol, more preferably 1-propanol, and
a palladium catalyst, to give the dilithium salt of Valsartan of formula (I):

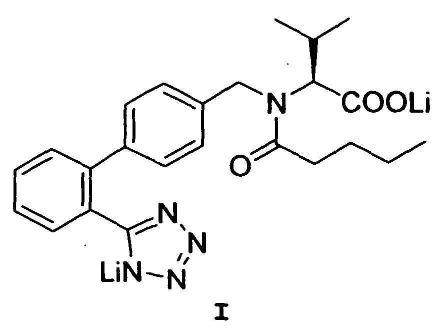

Surprisingly, selecting a lithium carbonate or lithium hydroxide or a hydrate thereof, preferably lithium hydroxide, as a base for carrying out the Suzuki coupling greatly facilitates the purification of the crude product obtained, owing to the fact that this crude product is purer than when sodium hydroxide or a derivative thereof is used.

This coupling can take place in homogeneous catalysis, if the palladium species is soluble in the reaction medium, as in the case of Pd (PPh₃)₄, PdCl₂(PPh₃)₂, Pd(AcO)₂, or heterogeneous if the palladium species is not soluble in the reaction medium, as in the case of Pd/C.

If Pd/C is used it is necessary to add catalytic amounts of a ligand, preferably of phosphine type, especially triphenylphosphine, or a water-soluble phosphine such as the trisodium salt of 3,3',3" - phosphinidinatris(benzenesulphonic) acid. The latter has the advantage of being soluble in water and, therefore, the reaction could be carried out in water as solvent.

The temperature of the reaction is between 25 and 150°C. Preferably, between 50 and 100°C in order to obtain the dilithium salt of Valsartan (I), or a hydrate or solvate thereof, from the intermediate products **II** and **III.**

The preparation of intermediate **II** is described in the published Spanish patent applications ES2251292 and ES200502558, and that of the intermediate **III** is described in patent DE4313737.

Under these conditions, the dilithium salt of Valsartan or a hydrate or solvate thereof is obtained.

If it is desired to purify the dilithium salt of Valsartan **(I),** or a hydrate or solvate thereof, the above crude product is suspended in acetonitrile and left under stirring. The resulting solid is filtered and is characterised as the trihydrate of the dilithium salt of Valsartan, which hereinafter will be designated as Form I of the dilithium salt of Valsartan characterised as trihydrate.

The present invention also relates to the use of said dilithium salt for obtaining Valsartan, useful for manufacturing a drug for treating hypertension and heart failure.

Thus, from the purified or unpurified dilithium salt of Valsartan **(I)** the Valsartan is extracted from the acid aqueous phase, preferably at a pH between 3 and 6, more preferably at a pH between 4 and 5, using an organic solvent not miscible in water such as ethyl acetate or isopropyl acetate, among others. Precipitation is then carried out by adding cyclohexane, which produces wet crude Valsartan.

The wet crude Valsartan is crystallised by using ethyl acetate and cyclohexane, preferably at a temperature between 20 and 100°C, more preferably between 30 and 70°C, in order to obtain the wet crystallised Valsartan, with a high yield and very high purity.

Surprisingly, in the present invention the Valsartan is obtained with a high yield and without the need of purification by means of silica gel column chromatography.

The product obtained from the preceding process can occlude solvent. Even after the standard drying processes high residual levels of solvents remain, which is unacceptable in an active ingredient of a pharmaceutical formulation, due to the usual toxicity of most organic solvents.

In order to remove them a process has been developed consisting of keeping the product in a dryer under stirring, at reduced pressure and with a stream of an inert gas such as nitrogen, at between 20 and 60°C for between 1 and 24 hours, being possible to vary the temperature with the time.

In this way, a dry and essentially amorphous Valsartan is obtained, as described in patent WO2002/006253, with solvent levels acceptable for a pharmaceutical formulation in accordance with ICH Q3C regulations.

There follows a description of some examples that show, by way of non-restrictive illustration of the present invention, preferred embodiments of the invention.

### EXAMPLES

### Example 1: Obtaining the dilithium salt of 3-methyl-(2S)-{pentanoyl-[2'-(1H-tetrazole-5-il)-biphenyl-4-ilmethyl]-amino}-butyric acid, Valsartan

To a stirred solution of 1000 g (2.7 mol) of (25)-[(4-bromo-benzyl)-pentanoyl-amino]-3-methylbutyric acid **(II),** 610 g (2.7 mol) of 2-(1*H*-tetrazole-5-il)-phenylboronic acid **(III),** 13.5 g (0.052 mol) of triphenylphosphine and 100 g (0.024 mol) of Pd/C in 4.5 L of 1-propanol and 0.5 L of water 453 g (10.8 mol) of monohydrated lithium hydroxide are added, which is then bubbled with nitrogen and refluxed for 6 hours. It is then cooled at between 20 and 30°C and 3.0 L of 1-propanol is added thereto and the catalyst is removed by means of a filtering, and the 1-propanol is removed at reduced pressure, thereby obtaining a residue that mostly contains the dilithium salt of Valsartan.

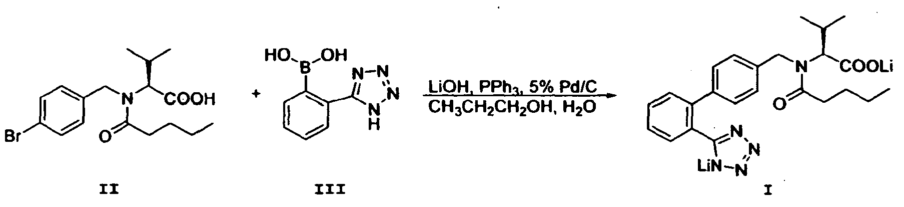

### Example 2: Obtaining the dilithium salt of 3-methyl-(2S)-{pentanoyl-[2'-(1H-tetrazole-5-il)-biphenyl-4-ilmethyl]-amino}-butyric acid, Valsartan

To a mixture made up of 0.54 L of ethanol, 5.4 L of 1,2-dimethoxyethane and 1.6 L of water 1000 g (2.70 mol) of (2S)-[(4-bromo-benzyl)-pentanoyl-amino)-3-methylbutyric acid **(II),** 510 g (2.68 mol) of 2-(1*H-*tetrazole-5-il)-phenylboronic acid **(III),** 156 g (0.135 mol) of tetrakistriphenylphosphine palladium and 453 g (10.8 mol) of monohydrated lithium hydroxide are added, which is bubbled with nitrogen and refluxed for 12 hours. It is then cooled at between 20 and 30°C, and 5 L of ethyl acetate and 5 L of water are added. The aqueous phase is decanted and it is extracted again with 5 L of ethyl acetate. The water is evaporated off, thus providing a residue that mostly contains the dilithium salt of Valsartan.

### Example 3: Purification of the dilithium salt of 3-methyl-(2S)-{pentanoyl-[2'-(1H-tetrazole-5-il)-biphenyl-4-ilmethyl]-amino}-butyric acid, Valsartan

The residue of Example 1 or 2 is suspended in acetonitrile and stirred overnight. The resulting white solid is filtered and characterised as Form I of the dilithium salt of Valsartan characterised as trihydrate.
IR (ATR): 3700-3000, 2958, 1603, 1459, 1410, 750 cm⁻¹.
[α]_{D}: -64 . 81 (c 1.0. methanol).
KF= 11.86% water.
DSC (10.0°C/min): 137.5 and 157.2°C.
XRPD: according to Table 1.

### Example 4: Crystallisation of 3-methyl-(2S)-{pentanoyl-[2'-(1H-tetrazole-5-il)-biphenyl-4-ilmethyl]-amino}-butyric acid, Valsartan

The residue of example 1, 2 or 3 is dissolved in 7.0 L of water and is extracted with ethyl acetate (2.5 L and 1.0 L), adjusting the pH of the aqueous phase to 4.0-4.2 at each extraction with a solution of 3N HCl. The organic phases are combined, heated with activated charcoal, filtered and the solvent evaporated off at reduced pressure.

Then 7.0 L of ethyl acetate is added and the mixture heated at 40-45°C with vigorous stirring and precipitated with 7.0 L of cyclohexane. It is left to cool at 20-25°C and then at 0°C, filtered and washed with a cold mixture of cyclohexane:ethyl acetate 1:1. This process is repeated once more.

This provides 1800 g of wet crystallised Form I Valsartan **(I)** that can contain up to 60% by weight of solvents.

The crude wet crystallised Valsartan is placed in a dryer with stirring at reduced pressure and with stream of an inert gas such as nitrogen, at between 20 and 60°C for between 1 and 24 hours, being possible to vary the temperature with the time.

Thus 826 g (70%, 1.9 mol) of Valsartan **(I)** with solvent levels acceptable for pharmaceutical formulation in accordance with ICH Q3C regulations is obtained.

## Claims

1. Dilithium salt of Valsartan of formula (I): 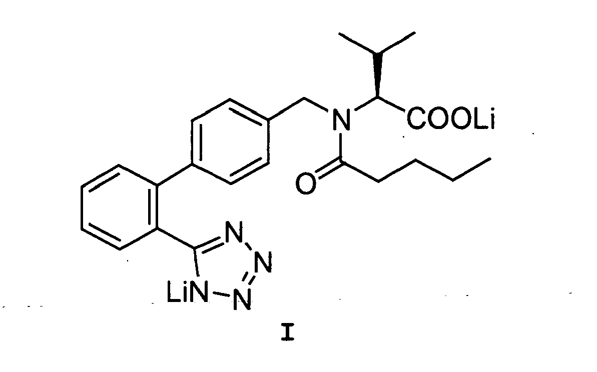 its polymorphs and solvates or hydrates thereof.

2. Polymorph of the dilithium salt of Valsartan according to Claim 1, designated as Form I of the dilithium salt of Valsartan **characterised** as trihydrate, **characterised by** a powder X-ray diffractogram, with peaks, in units of 2θ and d-spacing:
| Pos, 2θ | d-spacing, Å |
|---|---|
| 5.4 | 16.4042 |
| 10.7 | 8.2357 |
| 13.6 | 6.5069 |
| 14.4 | 6.1603 |
| 15.6 | 5.6881 |
| 16.1 | 5.4943 |
| 16.4 | 5.4120 |
| 17.1 | 5.1750 |
| 18.6 | 4.7612 |
| 21.0 | 4.2288 |
| 21.6 | 4.1101 |
| 23.1 | 3.8572 |
| 30.2 | 2.9585 |

3. Polymorph according to Claim 2, designated as Form I of the dilithium salt of Valsartan **characterised** as trihydrate, **characterised by** a DSC (10.0 °C/min) with two minimums, one at 137.5°C and a second minimum at 157.2°C

4. Polymorph of the dilithium salt of Valsartan according to Claim 2, designated as Form I of the dilithium salt of Valsartan **characterised** as trihydrate, **characterised by** an IR (ATR): 3700-3000, 2958, 1603, 1459, 1410, 750 cm⁻¹.

5. Process for obtaining the dilithium salt of Valsartan (I), comprising:
i) Coupling of the intermediate (II) with the 2-(1*H*-tetrazole-5-il)-phenylboronic acid of formula (III): 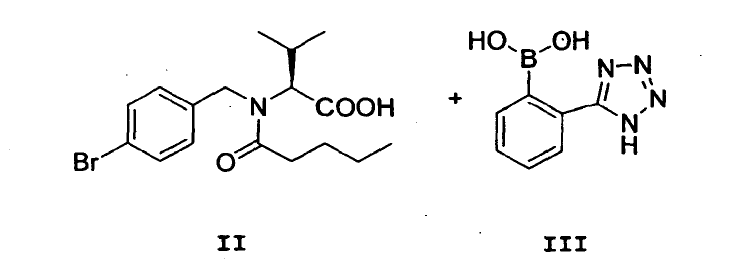
**characterised in that** said coupling takes place in the presence of a lithium base, and
a mixture of water and water-miscible organic solvent, and
a palladium catalyst, to give the dilithium salt of Valsartan of formula (I).

6. Process according to Claim 5, wherein said lithium base is selected from lithium carbonate or lithium hydroxide or a hydrate or solvate of these bases.

7. Process according to Claim 6, wherein said lithium base is monohydrated lithium hydroxide.

8. Process according to Claim 5, wherein the water-miscible organic solvent is an alcohol selected from methanol, ethanol, 1-propanol and 2-propanol.

9. Process according to Claim 8, wherein said alcohol is 1-propanol.

10. Process according to Claim 5, **characterised in that** from the dilithium salt of Valsartan (**I**) the acid form of Valsartan (I) is obtained by acidification of the mixture by isolating or without isolating said dilithium salt of Valsartan.

11. Use of the dilithium salt of Valsartan of formula (I), its polymorphs and solvates or hydrates thereof for obtaining Valsartan without the need of purification by silica gel column chromatography.

## Patentansprüche

1. Dilithiumsalz von Valsartan mit der Formel (I): 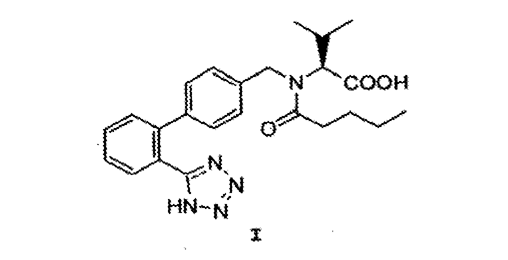 seine Polymorphe und Solvate oder Hydrate hiervon.

2. Polymorph des Dilithiumsalzes von Valsartan nach Anspruch 1, bezeichnet als Form I des Dilithiumsalzes von Valsartan, **gekennzeichnet** als Trihydrat, **gekennzeichnet durch** ein Röntgen-Pulverdiffraktogram mit Peaks, in Einheiten von 2θ und d-spacing:
| Pos, 2θ | d-spacing, Å |
|---|---|
| 5,4 | 16,4042 |
| 10,7 | 8,2357 |
| 13,6 | 6,5069 |
| 14,4 | 6,1603 |
| 15,6 | 5,6881 |
| 16,1 | 5,4943 |
| 16,4 | 5,4120 |
| 17,1 | 5,1750 |
| 18,6 | 4,7612 |
| 21,0 | 4,2288 |
| 21,6 | 4,1101 |
| 23,1 | 3,8572 |
| 30,2 | 2,9585 |

3. Polymorph nach Anspruch 2, bezeichnet als Form I des Dilithiumsalzes von Valsartan, **gekennzeichnet** als Trihydrat, **gekennzeichnet durch** ein DSC (10,0 °C/Min) mit zwei Minima, eines bei 137,5°C und ein zweites Minimum bei 157,2°C.

4. Polymorph des Dilithiumsalzes von Valsartan nach Anspruch 2, bezeichnet als Form I des Dilithiumsalzes von Valsartan **gekennzeichnet** als Trihydrat, **gekennzeichnet durch** ein IR (ATR): 3700-3000, 2958, 1603, 1459, 1410, 750 cm⁻¹.

5. Verfahren zur Herstellung des Dilithiumsalzes von Valsartan (I), umfassend:
i) Verknüpfen des Zwischenprodukts (II) mit der 2-(1*H*-Tetrazol-5-il)-phenylboronsäure der Formel (III): 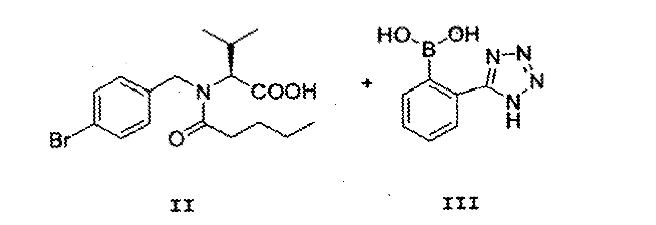
**dadurch gekennzeichnet, dass** das Verknüpfen in Gegenwart einer Lithiumbase stattfindet, und
einer Mischung aus Wasser und einem mit Wasser mischbaren organischen Lösungsmittel, und
einem Palladiumkatalysator, um das Dilithiumsalz von Valsartan der Formel (I) zu erhalten.

6. Verfahren nach Anspruch 5, wobei die Lithiumbase ausgewählt wird aus Lithiumkarbonat oder Lithiumhydroxyd oder einem Hydrat oder Solvat dieser Basen.

7. Verfahren nach Anspruch 6, wobei die Lithiumbase monohydriertes Lithiumhydroxyd ist.

8. Verfahren nach Anspruch 5, wobei das mit Wasser mischbare organische Lösungsmittel ein Alkohol ist, ausgewählt aus Methanol, Ethanol, 1-Propanol und 2-Propanol.

9. Verfahren nach Anspruch 8, wobei der Alkohol 1-Propanol ist.

10. Verfahren nach Anspruch 5, **dadurch gekennzeichnet, dass** aus dem Dilithiumsalz von Valsartan (I) die Säureform von Valsartan (I) durch Ansäuerung der Mischung erhalten wird, durch Isolieren oder ohne Isolieren des Dilithiumsalzes von Valsartan.

11. Verwendung des Dilithiumsalzes von Valsartan der Formel (I), seiner Polymorphe und Solvate oder Hydrate hiervon, um Valsartan ohne die Notwendigkeit einer Reinigung durch eine Silicagelsäulenchromatographie zu erhalten.

## Revendications

1. Sel de dilithium de Valsartan de formule (I) : 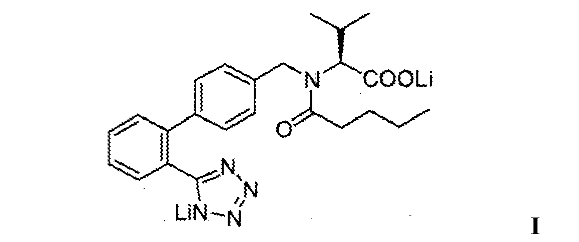 ses formes polymorphiques et leurs solvates ou hydrates.

2. Forme polymorphique du sel de dilithium de Valsartan selon la revendication 1, désignée sous le nom de Forme I du sel de dilithium de Valsartan **caractérisée** sous forme de trihydrate, **caractérisée par** un diagramme de diffraction des rayons X réalisé sur une poudre, présentant les pics, en unités de 2θ et les distances d :
| Position, 2θ | Distance d, Å |
|---|---|
| 5,4 | 16,4042 |
| 10,7 | 8,2357 |
| 13,6 | 6,5069 |
| 14,4 | 6,1603 |
| 15,6 | 5,6881 |
| 16,1 | 5,4943 |
| 16,4 | 5,4120 |
| 17,1 | 5,1750 |
| 18,6 | 4,7612 |
| 21,0 | 4,2288 |
| 21,6 | 4,1101 |
| 23,1 | 3,8572 |
| 30,2 | 2,9585 |

3. Forme polymorphique selon la revendication 2, désignée sous le nom de Forme I du sel de dilithium de Valsartan **caractérisée** sous forme de trihydrate, **caractérisée par** un spectre DSC (10,0°C/min) présentant deux minimums, un à 137,5°C et un second minimum à 157,2°C.

4. Forme polymorphique du sel de dilithium de Valsartan selon la revendication 2, désignée sous le nom de Forme I du sel de dilithium de Valsartan **caractérisée** sous forme de trihydrate, **caractérisée par** un spectre IR (ATR) : 3700-3000, 2958, 1603, 1459, 1410, 750 cm⁻¹.

5. Procédé de préparation du sel de dilithium de Valsartan (I), comprenant :
i) le couplage de l'intermédiaire (II) avec l'acide 2-(1H-tétrazole-5-yl)phényl boronique de formule (III) : 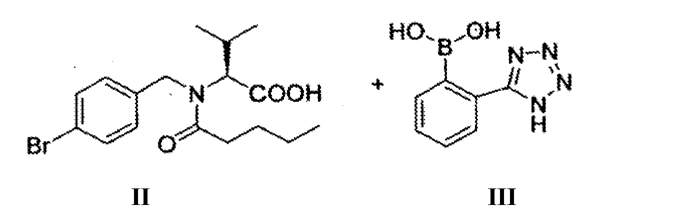
**caractérisé en ce que** ledit couplage s'effectue en présence d'une base lithiée, et
d'un mélange d'eau et d'un solvant organique miscible à l'eau, et
d'un catalyseur au palladium, pour donner le sel de dilithium de Valsartan de formule (I).

6. Procédé selon la revendication 5, dans lequel ladite base lithiée est choisie parmi le carbonate de lithium et l'hydroxyde de lithium et un hydrate ou un solvate de ces bases.

7. Procédé selon la revendication 6, dans lequel ladite base lithiée est l'hydroxyde de lithium monohydrate.

8. Procédé selon la revendication 5, dans lequel le solvant organique miscible à l'eau est un alcool choisi parmi le méthanol, l'éthanol, le 1-propanol et le 2-propanol.

9. Procédé selon la revendication 8, dans lequel ledit alcool est le 1-propanol.

10. Procédé selon la revendication 5, **caractérisé en ce qu'**à partir du sel de dilithium de Valsartan (I), la forme acide du Valsartan (I) est obtenue par acidification du mélange, avec isolement ou sans isolement dudit sel de dilithium de Valsartan.

11. Utilisation du sel de dilithium de Valsartan de formule (I), de ses formes polymorphiques et de leurs solvates ou hydrates pour obtenir le Valsartan sans nécessité d'une purification par chromatographie sur colonne de gel de silice.
